# EUROPEAN PATENT SPECIFICATION

(11) **EP 1 682 488 B1**
(45) Date of publication and mention of the grant of the patent: **02.01.2013**
(21) Application number: 04804523.1
(22) Date of filing: 09.11.2004
(51) Int. Cl.: C07C 231/02, C07C 231/24, C07C 233/08

(54) **PROCESS FOR THE PREPARATION OF GABAPENTIN**
VERFAHREN ZUR HERSTELLUNG VON GABAPENTIN
PROCEDE DE PREPARATION DE LA GABAPENTINE

(30) Priority: 11.11.2003 IT MI20032165
(43) Date of publication of application: 26.07.2006
(73) Proprietor: ZaCh System S.p.A., 20091 Bresso (Milano) (IT)
(72) Inventor: ARRIGHI, Katiuscia, I-20020 Misinto (IT); CANNATA, Vincenzo, I-40037 Sasso Marconi (IT); CORCELLA, Francesco, I-20015 Parabiago (IT); MARCHIORO, Gaetano, I-36100 Vicenza (IT); NICOLI, Andrea, I-36100 Vicenza (IT); PAIOCCHI, Maurizio, I-20152 Milano (IT); VILLA, Marco, I-20125 Milano (IT)
(86) International application number: PCT/EP2004/052894
(87) International publication number: WO 2005/044779

(56) References cited:
- WO-A-02/34709
- WO-A-03/002517
- DATABASE WPI Section Ch, Week 200155 Derwent Publications Ltd., London, GB; Class B05, AN 2001-497525 XP002325720 & CN 1 297 885 A (HANGZHOU SHOUXIN FINE CHEM CO LTD) 6 June 2001 (2001-06-06) cited in the application

## Description

The present invention relates to an improved process for the preparation of gabapentin and, more particularly, to an improvement of the preparation reaction of 1,1-cyclohexanediacetic acid monoamide, intermediate utilized in the preparation of gabapentin.

Gabapentin, 1-(aminomethyl)-cyclohexaneacetic acid (The Merck Index, XII Ed., page 733, n° 4343), is a known drug with anti-epileptic and anticonvulsant activity described for the first time in the US patent No. 4.024.175 in the name of Wamer-Lambert Co.

In the literature several processes for the preparation of gabapentin are reported, see for example the already mentioned US patent No. 4.024.175, the US patents Nos. 5.068.413 and 5.091.567 both in the name of Gödecke A.G.

The US patent 4.024.175 describes various processes for the preparation of gabapentin or analogous compounds of formula 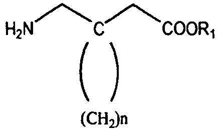 wherein R₁ is a hydrogen atom or a lower alkyl and n is 4, 5 or 6;
characterized in that they use conventional methods for the preparation of primary amines or aminoacids such as, for example, the Curtius rearrangement of appropriate azides, the Hofmann rearrangement of appropriate monoamides or the Lossen rearrangement of appropriate hydroxamic acids.

In particular, the patent mentioned above in the name of Warner-Lambert Co., Example 4 variant A, column 5, describes the synthesis of the lower cyclic homologous derivative of gabapentin, 1-(methylamino)-1-cyclopentaneacetic acid, through the preparation of cyclopentanediacetic acid monoamide, carried out by reaction of the corresponding anhydride with an aqueous solution of 20% NH₃, the Hofmann rearrangement of the obtained monoamide, the acidification and the extraction followed by a final purification step consisting in the elution through a basic ion exchange resin and in the recrystallization from alcohols.

In the patent CN 1297885 (Hangzhou Shouxin Fine Chem) [abstract taken from World Patent Index (online), Derwent Publications, London, Accession n° 2001-497525], the preparation of the 1,1-cyclohexyl monoamide of oxalic acid is described through the reaction of the corresponding anhydride with aqueous or gaseous ammonia in the presence of an organic solvent.

Known these synthetic techniques, the International patent application WO 03/002517 in the name of Bromine Compounds describes a process for the synthesis of 1,1-cyclohexanediacetic acid monoamide comprising:
a) the amination of the anhydride of 1,1-cyclohexanediacetic acid with aqueous ammonia.
b) the neutralization of the reaction mixture, therethrough the crude 1,1-cyclohexanediacetic acid monoamide is precipitated and filtered.
c) the purification of the crude 1,1-cyclohexanediacetic acid monoamide through a crystallization from solvent.

Although the process mentioned above can be considered an attempt of transforming on industrial scale the laboratory process described in the Warner Lambert patent, nevertheless it appears to be a not much effective process from the industrial point of view.

In particular, it utilizes a considerable amount of reagents and solvents. For example, the crystallization step provides a great use of solvents and furthermore the amination requires a considerable amount of ammoniacal solution which has to be disposed and this constitutes extra costs and disposal time.

Furthermore, in the patent 1,1-cyclohexanediacetic acid monoamide is described and claimed with a purity higher than 99.5% (besides already obtainable according to what reported in the abstract of the Chinese patent CN 1297885, already cited) and above all such product purity is obtained to the detriment of process time and costs without being necessary for the transformations thereto the product in the gabapentin synthesis is subjected. Consequently, it becomes necessary to study alternative methodologies allowing the implementation of the reaction under more favourable conditions from the point of view of the industrial application of the process.

Now, we have surprisingly found improved reaction conditions for the synthesis of 1,1-cyclohexanediacetic acid monoamide, intermediate in the gabapentin preparation, at industrial level which allow to overcome the drawbacks shown by the processes described by the known art.

Therefore, object of the present invention is a process for the synthesis of gabapentin comprising the preparation of 1,1-cyclohexanediacetic acid monoamide, the Hofmann transposition of the same monoamide, the purification of a gabapentin salt and the crystallization from organic solvent, characterized in that the preparation of the acid monoamide comprises:
a) the amination of 1,1-cyclohexanediacetic acid anhydride by reaction with aqueous NH₃ at a temperature lower than 30°C by using a NH₃/anhydride molar ratio lower than 3.
b) the product precipitation through the acidification of the reaction mixture.

The anhydride of 1,1-cyclohexanediacetic acid is prepared according to known techniques, for example, according to the method described in the French patent FR 1.248.764 in the name of Centre de Lyophilisation Pharmaceutique or in Callahan et al., J. Org. Chem., 1988, vol. 53, 1527-1530.

Generally, the transformation of 1,1-cyclohexanediacetic acid into the corresponding anhydride is carried out by reaction with acetic anhydride in the presence of an organic solvent commonly utilized in the industrial processes.

Specific examples of utilized organic solvents are methyl ter-butyl ether, toluene, tetrahydrofuran and methylene chloride.

Preferably, the transformation of 1,1-cyclohexanediacetic acid into the corresponding anhydride is carried out by reaction with acetic anhydride in the presence of toluene.

The amination takes place by reaction with NH₃ generally utilized in aqueous solution with a concentration comprised between 25 and 35% and preferably with ammonia in aqueous solution with a concentration around 28%.

The acidification step is carried out by using common organic and inorganic acids such as, for example, hydrochloric, hydrobromic, hydriodic, nitric, sulfuric, phosphoric, carbonic, acetic, tartaric, citric, benzoic, maleic, fumaric, succinic, glutaric, metansulfonic, benzensulfonic, paratoluensulfonic, trichloroacetic and trifluoroacetic acid.

The organic and inorganic acids are usually utilized in aqueous solution, but some of them can be used in the gaseous phase.

The acidification step is preferably carried out with concentrated or gaseous hydrochloric acid and still more preferably with aqueous hydrochloric acid with a concentration around 31%.

The molar ratio between ammonia and 1,1-cyclohexanediacetic acid anhydride is generally comprised between 2.2 and 2.9 and preferably between 2.5 and 2.7 in order to optimize the yield and limit the scraps.

Keeping the temperature below 30°C during the amination reaction allows reducing to the minimum the impurity formation.

From a practical point of view one proceeds in adding anhydride into a reactor, containing the ammoniacal solution, thermostated at a temperature lower than 30°C and preferably at a temperature comprised between 10 and 25°C.

The acidification step therewith 1,1-cyclohexanediacetic acid monoamide is precipitated constitutes a critical aspect as well as an additional object of the present invention. This precipitation method consists in acidifying at a temperature comprised between 40 and 45°C until obtaining pH 6.3-6.5, (the crystal is left to enlarge) and then continuing to acidify at the same temperature until obtaining pH 3.8-4.2 optimum for the precipitation and, at last, the precipitate is filtered by keeping the temperature at about 40-45°C.

Therefore, a second object of the present invention is the precipitation method of 1,1-cyclohexanediacetic acid monoamide comprising the acidification of an ammoniacal solution of the monoamide at a temperature comprised between 40 and 45°C until obtaining a pH value 6.3-6.5, the continuation of the acidification step of the reaction mixture at the same temperature until obtaining a pH value 3.8-4.2 and, at last, the filtration of the precipitate by keeping the temperature between 40 and 45°C.

By acting through the improved method, object of the present invention, a highly pure product is obtained (purity not lower than 99%, suitable for the subsequent steps of gabapentin preparation) with very high yields (about 95%) and above all the crystallization process of the product itself is made unnecessary.

It is clear to the person skilled in the art how it is obvious, in order to obtain a higher product purity, to purify 1,1-cyclohexanediacetic acid monoamide for example by crystallization, nevertheless this additional expensive step in the process does not lead to any industrial advantage.

The process object of the present invention allows obtaining 1,1-cyclohexanediacetic acid monoamide with a smaller number of synthetic steps and, consequently, in a reduced time and with reduced costs.

Furthermore, the use of reagents and solvents is greatly limited with additional advantages under the industrial scrap disposal point of view.

In fact, the improvement in the preparation of 1,1-cyclohexanediacetic acid monoamide in the gabapentin synthesis, object of the present invention, allows obtaining a product which has analogous, if not better, features compared to the one obtained with the known method, the process is more effective with a low consumption in ammonia and without the need of purifying the product by crystallization.

From the comparison with the known art, in particular with reference to the International patent application WO 03/02517, already mentioned, some substantial differences can be then pointed out:
- use of a lower quantity of NH₃ (NH₃/anhydride molar ratio lower than 3 whereas the prior art mentioned above utilizes a molar ratio comprised between 5 and 10, preferably equal to 7);
- no need to crystallize 1,1-cyclohexanediacetic acid monoamide, obtaining a product suitable to be used in the subsequent steps of the gabapentin preparation. Consequently, the process provides one industrial step less with respect to the prior art with all the advantages coming therefrom such as, for example, shorter implementation time, reduced use of solvents, less use of manpower, less occupation of reactors, etc.

Furthermore, the process yield is much higher than the one shown in the examples of the patent application mentioned above in the name of Bromine Compounds.

A practical embodiment of the process object of the present invention provides the transformation of 1,1-cyclohexanediacetic acid in the corresponding anhydride by reaction with acetic anhydride in toluene. After having removed by distillation the greater part of acetic acid which has formed and part of toluene, the intermediate dissolved into toluene is added to aqueous ammonia solution. Toluene is eliminated by separation of the phases and the monoamide is isolated by centrifugation of the acid aqueous solution. Then, one proceeds in transforming the obtained product into gabapentin, for example, through the Hofmann rearrangement followed by a purification step by column chromatography through ionic exchange resins of the obtained gabapentin salt and the crystallization from alcoholic solvents.

In order to better illustrated the invention, the following example is now provided.

### Example 1

3246 kg (3748 1) of toluene and in nitrogen flow and under stirring 1874 kg of 1,1-cyclohexanediacetic acid were charged in a reactor.

A dense suspension was obtained. The suspension was heated at 80°C and 1146 kg (1064 1) of acetic anhydride were added thereto in 2-3 hours.

The addition was slightly endothermic. During the addition the inner temperature was kept at about 80°C.

Upon proceeding with the addition the reaction mixture fluidified until complete dissolution The mixture was left under stirring for about 30 min. at about 80°C inside, then it was gradually vacuum-placed and it was distilled by keeping the inner temperature below 80°C until a residue volume of about 2600 1.

About 3800 kg of a mixture, about 25/75 w/w acetic acid/toluene, which were sent to the incinerator, were distilled.

The distillation residue crystallized at a temperature of about 40-50°C, then it was kept dissolved at the temperature of 50-60°C.

In the meantime in a second reactor an ammoniacal solution was prepared by charging 656 kg of demineralized water and 1500 kg (1670 1) of an ammonia solution, about 28%, were added thereto.

By keeping the inner temperature at 10-25°C the distillation residue previously obtained and kept dissolved at 50-60°C (the addition was exothermic) was added.

The pH was controlled which have to remain higher than a value of 8 during and at the end of the addition.

The obtained biphasic solution was stirred for about 20 minutes at 20-30°C, then it was left to decant for one how.

The lower aqueous phase was separated, at room temperature, whereas the toluenic phase was sent to the incenerator.

The aqueous phase was gradually vacuum-placed to remove possible toluene and ammonia traces.

To the aqueous solution 3000 kg of demineralized water were added and the inner temperature was brought to 40-45°C.

Then, by keeping the inner temperature at 40-45°C, about 1596 kg (1386 1) of hydrochloric acid in solution were added.

It was left under stirring by still keeping the inner temperature of 40-05°C until obtaining pH 3.8-4.2. At the end of the addition it was stirred for about 20 minutes and the pH was controlled again.

By keeping the temperature at 40-45°C it was filtered and each filtration washed with four washings each one constituted by about 255 kg of demineralized water.

About 2000 kg of wet product were obtained which was sent to the drying.

The process yield was higher than 95%.

The titre of the reaction product evaluated by means of the HPLC method was greater thaw 99% (total unknown impurities lower than 0.1%).

The resultant 1,1-cyclohexanediacetic acid monoamide was transformed into gabapentin through known methods, for example, by the Hofmann rearrangement, the acidification, extraction, purification of an aqueous solution of gabapentin hydrochloride on a strong cationic ion exchange resin followed by recrystallization as described in the International patent application WO 02/34709 in the name of the same applicant.

## Claims

1. A process for the synthesis of gabapentin comprising the preparation of 1,1-cyclohexanediacetic acid monoamide, the Hofmann transposition of the same monoamide, the purification of a gabapentin salt and the crystallization from organic solvent, **characterized in that** the preparation of the acid monoamide comprises:
a) the amination of 1,1-cyclohexanediacetic acid anhydride by reaction with aqueous NH₃ at a temperature comprised between 10 and 25°C by using a NH₃/nhydride molar ratio lower than 3;
b) the precipitation of 1,1-cyclohexanediacetic acid monoamide comprising the acidification of an ammoniacal solution of the monoamide obtained in step a) at a temperature comprised between 40 and 45°C until obtaining a pH value 6.3-6.5, the continuation of the acidification step of the reaction mixture at the same temperature until obtaining a pH value 3.8-4.2 and, at last, the precipitate filtration by keeping the temperature between 40 and 45°C.

2. A process according to claim I wherein the amination of 1,1-cyclohexanediacetic acid anhydride takes place by reaction with NH₃ utilized in aqueous solution with a concentration comprised between 25 and 35%.

3. A process according to claim 2 wherein the amination of 1,1-cyclohexanediacetic acid anhydride takes place by reaction with ammonia in aqueous solution with a concentration 28%.

4. A process according to claim 1 wherein the acidification step is carried out with concentrated or gaseous hydrochloric acid.

5. A process according to claim 4 wherein the acidification step is carried out with aqueous hydrochloric acid with a concentration 31%.

6. A process according to claim 1 wherein the molar ratio between ammonia and 1,1-cyclohexanediacetic acid anhydride is comprised between 2.2 and 2.9.

7. A process according to claim 6 wherein the molar ratio between ammonia and 1,1-cyclohexanediacetic acid anhydride is comprised between 2.5 and 2.7.

8. A precipitation process of 1,1-cyclohexanediacetic acid monoamide comprising the acidification of an ammoniacal solution of the monoamide at a temperature comprised between 40 and 45°C until obtaining a pH value 6.3-6.5, the continuation of the acidification step of the reaction mixture at the same temperature until obtaining a pH value 3.8-4.2 and, at last, the precipitate filtration by keeping the temperature between 40 and 45°C.

9. A process according to claim 8 wherein the acidification step is carried out with concentrated or gaseous hydrochloric acid.

10. A process according to claim 9 wherein the acidification step is carried out with aqueous hydrochloric acid with a concentration 31%.

11. A process according to claim I further comprising the transformation of 1,1-cyclohexanediacetic acid into the corresponding anhydride.

12. A process according to claim 11 wherein the transformation of 1,1-cyclohexanediacetic acid into the corresponding anhydride is carried out by reaction with acetic anhydride in the presence of an organic solvent.

13. A process according to claim 12 wherein the organic solvent is toluene.

14. A process for the preparation of 1,1-cyclohexanediacetic acid monoamide comprising:
a) the amination of 1,1-cyclohexanediacetic acid anhydride by reaction with aqueous NH₃ at a temperature comprised between 10 and 25°C by using a NH₃/anhydride molar ratio lower than 3;
b) the precipitation of 1,1-cyclohexanediacetic acid monoamide comprising the acidification of an ammoniacal solution of the monoamide obtained in step a) at a temperature comprised between 40 and 45°C until obtaining a pH value 6.3-6.5, the continuation of the acidification step of the reaction mixture at the same temperature until obtaining a pH value 3.8-4.2 and, at last, the precipitate filtration by keeping the temperature between 40 and 45°C.

15. A process according to claim 14 wherein the amination of 1,1-cyclohexanediacetic acid anhydride takes place by reaction with NH₃ generally utilized in aqueous solution with a concentration comprised between 25 and 35%.

16. A process according to claim 15 wherein the amination of 1,1-cyclohexanediacetic acid anhydride takes place by reaction with ammonia in aqueous solution with a concentration 28%.

17. A process according to claim 14 wherein the acidification step is carried out with concentrated or gaseous hydrochloric acid.

18. A process according to claim 17 wherein the acidification step is carried out with aqueous hydrochloric acid with a concentration 31%.

19. A process according to claim 14 wherein the molar ratio between ammonia and 1,1-cyclohexanediacetic acid anhydride is comprised between 2.2 and 2.9.

20. A process according to claim 19 wherein the molar ration between ammonia and 1,1-cyclohexanediacetic acid anhydride is comprised between 2.5 and 2.7.

21. A process according to claim 14 further comprising the transformation of 1,1-cyclohexanediacetic acid into the corresponding anhydride.

22. A process according to claim 21 wherein the transformation of 1,1-cyclohexanediacetic acid into the corresponding anhydride is carried out by reaction with acetic anhydride in the presence of an organic solvent.

23. A process according to claim 22 wherein the organic solvent is toluene.

## Patentansprüche

1. Verfahren zur Synthese von Gabapentin, umfassend die Herstellung von 1,1-Cyclohexandiessigsäuremonoamid, die Hofmann-Transposition desselben Monoamids, die Reinigung eines Gabapentinsalzes und die Kristallisation aus organischem Lösungsmittel, **dadurch gekennzeichnet, dass** die Herstellung des sauren Monoamids Folgendes umfasst:
a) die Aminierung von 1,1-Cyclohexandiessigsäureanhydrid durch Reaktion mit wässrigem NH₃ bei einer Temperatur, die zwischen 10 und 25 °C liegt, unter Anwendung eines Molverhältnisses von NH₃ : Anhydrid von weniger als 3;
b) das Ausfällen von 1,1-Cyclohexandiessigsäuremonoamid, die Ansäuerung einer ammoniakalischen Lösung des in Schritt a) erhaltenen Monoamids bei einer Temperatur, die zwischen 40 und 45 °C liegt, bis zum Erhalten eines pH-Werts von 6,3 - 6,5, die Fortsetzung des Ansäuerungsschritts der Reaktionsmischung bei derselben Temperatur bis zum Erhalten eines pH-Werts von 3,8 - 4,2 und schließlich das Ausfällungsfiltrieren durch Halten der Temperatur zwischen 40 und 45 °C umfassend.

2. Verfahren nach Anspruch 1, wobei die Aminierung von 1,1-Cyclohexandiessigsäureanhydrid durch Reagieren mit NH₃ erfolgt, das in wässriger Lösung mit einer Konzentration zwischen 25 und 35 % verwendet wird.

3. Verfahren nach Anspruch 2, wobei die Aminierung von 1,1-Cyclohexandiessigsäureanhydrid durch Reaktion mit Ammoniak in wässriger Lösung mit einer Konzentration von 28 % erfolgt.

4. Verfahren nach Anspruch 1, wobei der Ansäuerungsschritt mit konzentrierter oder gasförmiger Salzsäure durchgeführt wird.

5. Verfahren nach Anspruch 4, wobei der Ansäuerungsschritt mit wässriger Salzsäure mit einer Konzentration von 31 % durchgeführt wird.

6. Verfahren nach Anspruch 1, wobei das Molverhältnis zwischen Ammoniak und 1,1-Cyclohexandiessigsäureanhydrid zwischen 2,2 und 2,9 liegt.

7. Verfahren nach Anspruch 6, wobei das Molverhältnis zwischen Ammoniak und 1,1-Cyclohexandiessigsäureanhydrid zwischen 2,5 und 2,7 liegt.

8. Ausfällungsverfahren für 1,1-Cyclohexandiessigsäuremonoamid, umfassend das Ansäuern einer ammoniakalischen Lösung des Monoamids bei einer Temperatur, die zwischen 40 und 45 °C liegt, bis zum Erhalten eines pH-Werts von 6,3 - 6,5, die Fortsetzung des Ansäuerungsschritts der Reaktionsmischung bei derselben Temperatur bis zum Erhalten eines pH-Werts von 3,8 - 4,2 und schließlich das Ausfällungsfiltrieren durch Halten der Temperatur zwischen 40 und 45 °C.

9. Verfahren nach Anspruch 8, wobei der Ansäuerungsschritt mit konzentrierter oder gasförmiger Salzsäure durchgeführt wird.

10. Verfahren nach Anspruch 9, wobei der Ansäuerungsschritt mit wässriger Salzsäure mit einer Konzentration von 31 % durchgeführt wird.

11. Verfahren nach Anspruch 1, des Weiteren die Umwandlung von 1,1-Cyclohexandiessigsäure zu dem entsprechenden Anhydrid umfassend.

12. Verfahren nach Anspruch 11, wobei die Umwandlung von 1,1-Cyclohexandiessigsäure zu dem entsprechenden Anhydrid durch Reaktion mit Essigsäureanhydrid in Gegenwart eines organischen Lösungsmittels durchgeführt wird.

13. Verfahren nach Anspruch 12, wobei das organische Lösungsmittel Toluol ist.

14. Verfahren zur Herstellung von 1,1-Cyclohexandiessigsäuremonoamid, umfassend:
a) die Aminierung von 1,1-Cyclohexandiessigsäureanhydrid durch Reaktion mit wässrigem NH₃ bei einer Temperatur, die zwischen 10 und 25 °C liegt, unter Anwendung eines Molverhältnisses von NH₃ : Anhydrid von weniger als 3;
b) das Ausfällen von 1,1-Cyclohexandiessigsäuremonoamid, die Ansäuerung einer ammoniakalischen Lösung des in Schritt a) erhaltenen Monoamids bei einer Temperatur, die zwischen 40 und 45 °C liegt, bis zum Erhalten eines pH-Werts von 6,3 - 6,5, die Fortsetzung des Ansäuerungsschritts der Reaktionsmischung bei derselben Temperatur bis zum Erhalten eines pH-Werts von 3,8 - 4,2 und schließlich das Ausfällungsfiltrieren durch Halten der Temperatur zwischen 40 und 45 °C umfassend.

15. Verfahren nach Anspruch 14, wobei die Aminierung von 1,1-Cyclohexandiessigsäureanhydrid durch Reagieren mit NH₃ erfolgt, das im Allgemeinen in wässriger Lösung mit einer Konzentration, die zwischen 25 und 35 % liegt, verwendet wird.

16. Verfahren nach Anspruch 15, wobei die Aminierung von 1,1-Cyclohexandiessigsäureanhydrid durch Reaktion mit Ammoniak in wässriger Lösung mit einer Konzentration von 28 % erfolgt.

17. Verfahren nach Anspruch 14, wobei der Ansäuerungsschritt mit konzentrierter oder gasförmiger Salzsäure durchgeführt wird.

18. Verfahren nach Anspruch 17, wobei der Ansäuerungsschritt mit wässriger Salzsäure mit einer Konzentration von 31 % durchgeführt wird.

19. Verfahren nach Anspruch 14, wobei das Molverhältnis zwischen Ammoniak und 1,1-Cyclohexandiessigsäureanhydrid zwischen 2,2 und 2,9 liegt.

20. Verfahren nach Anspruch 19, wobei das Molverhältnis zwischen Ammoniak und 1,1-Cyclohexandiessigsäureanhydrid zwischen 2,5 und 2,7 liegt.

21. Verfahren nach Anspruch 14, des Weiteren die Umwandlung von 1,1-Cyclohexandiessigsäure in das entsprechende Anhydrid umfassend.

22. Verfahren nach Anspruch 21, wobei die Umwandlung von 1,1-Cyclohexandiessigsäure zu dem entsprechenden Anhydrid durch Reaktion mit Essigsäureanhydrid in Gegenwert eines organischen Lösungsmittels durchgeführt wird.

23. Verfahren nach Anspruch 22, wobei das organische Lösungsmittel Toluol ist.

## Revendications

1. Procédé pour la synthèse de gabapentine comprenant la préparation de monamide d'acide 1,1-cyclohexanediacétique, la transposition de Hofmann de ce monoamide, la purification d'un sel de gabapentine et la cristallisation à partir d'un solvant organique, **caractérisé en ce que** la préparation du monoamide comprend :
a) l'amination d'anhydride d'acide 1,1-cyclohexanediacétique par réaction avec une solution aqueuse de NH₃ à une température comprise entre 10 et 25 °C en utilisant un rapport molaire de NH₃/anhydride inférieur à 3 ;
b) la précipitation du monoamide d'acide 1,1-cyclohexanediacétique comprenant l'acidification d'une solution ammoniacale du monoamide obtenu dans l'étape a) à une température comprise entre 40 et 45 °C jusqu'à l'obtention d'une valeur de pH de 6,3 à 6,5, la poursuite de l'étape d'acidification du mélange réactionnel à la même température jusqu'à l'obtention d'une valeur de pH de 3,8 à 4,2, et enfin la filtration du précipité en maintenant la température entre 40 et 45 °C.

2. Procédé selon la revendication 1, dans lequel l'amination de l'anhydride d'acide 1,1-cyclohexanediacétique a lieu par réaction avec du NH₃ utilisé en solution aqueuse à une concentration comprise entre 25 et 35 %.

3. Procédé selon la revendication 2, dans lequel l'amination de l'anhydride d'acide 1,1-cyclohexanediacétique a lieu par réaction avec de l'ammoniac en solution aqueuse à une concentration de 28 %.

4. Procédé selon la revendication 1, dans lequel l'étape d'acidification est réalisée avec de l'acide chlorhydrique concentré ou gazeux.

5. Procédé selon la revendication 4, dans lequel l'étape d'acidification est réalisée avec une solution aqueuse d'acide chlorhydrique à une concentration de 31 %.

6. Procédé selon la revendication 1, dans lequel le rapport molaire entre l'ammoniac et l'anhydride d'acide 1,1-cyclohexanediacétique est compris entre 2,2 et 2,9.

7. Procédé selon la revendication 6, dans lequel le rapport molaire entre l'ammoniac et l'anhydride d'acide 1,1-cyclohexanediacétique est compris entre 2,5 et 2,7.

8. Procédé de précipitation du monoamide d'acide 1,1-cyclohexanediacétique comprenant l'acidification d'une solution ammoniacale du monoamide à une température comprise entre 40 et 45 °C jusqu'à l'obtention d'une valeur de pH de 6,3 à 6,5, la poursuite de l'étape d'acidification du mélange réactionnel à la même température jusqu' à l'obtention d' une valeur de pH de 3,8 à 4,2 et enfin la filtration du précipité en maintenant la température entre 40 et 45 °C.

9. Procédé selon la revendication 8, dans lequel l'étape d'acidification est réalisée avec de l'acide chlorhydrique concentré ou gazeux.

10. Procédé selon la revendication 9, dans lequel l'étape d'acidification est réalisée avec une solution aqueuse d'acide chlorhydrique à une concentration de 31 %.

11. Procédé selon la revendication 1, comprenant en outre la transformation de l'acide 1,1-cyclohexanediacétique en l'anhydride correspondant.

12. Procédé selon la revendication 11, dans lequel la transformation de l'acide 1,1-cyclohexanediacétique en 1"anhydride correspondant est réalisée par réaction avec de l'anhydride acétique en présence d'un solvant organique.

13. Procédé selon la revendication 12, dans lequel le solvant organique est le toluène.

14. Procédé pour la préparation du monoamide d'acide 1,1-cyclohexane-diacétique comprenant :
a) l'amination de l'anhydride d'acide 1,1-cyclohexanediacétique par réaction avec une solution aqueuse de NH₃ à une température comprise entre 10 et 25 °C en utilisant un rapport molaire de NH₃/anhydride inférieur à 3 ;
b) la précipitation du monoamide d'acide 1,1-cyclohexanediacétique comprenant l'acidification d'une solution ammoniacale du monoamide obtenu dans l'étape a) à une température comprise entre 40 et 45 °C jusqu'à l'obtention d'une valeur de pH de 6,3 à 6,5, la poursuite de l'étape d'acidification du mélange réactionnel à la même température jusqu'à l'obtention d'une valeur de pH de 3,8 à 4,2, et enfin la filtration du précipité en maintenant la température entre 40 et 45 °C.

15. Procédé selon la revendication 14, dans lequel l'amination de l'anhydride d'acide 1,1-cyclohexanediacétique a lieu par réaction avec du NH₃ utilisé en solution aqueuse à une concentration comprise entre 25 et 35 %.

16. Procédé selon la revendication 15, dans lequel l'amination de l'anhydride d'acide 1,1-cyclohexanediacétique a lieu par réaction avec de l'ammoniac en solution aqueuse à une concentration de 28 %.

17. Procédé selon la revendication 14, dans lequel l'étape d'acidification est réalisée avec de l'acide chlorhydrique concentré ou gazeux.

18. Procédé selon la revendication 17, dans lequel l'étape d'acidification est réalisée avec une solution aqueuse d'acide chlorhydrique à une concentration de 31 %.

19. Procédé selon la revendication 14, dans lequel le rapport molaire entre l'ammoniac et l'anhydride d'acide 1,1-cyclohexanediacétique est compris entre 2,2 et 2,9.

20. Procédé selon la revendication 19, dans lequel le rapport molaire entre l'ammoniac et l'anhydride d'acide 1,1-cyclohexanediacétique est compris entre 2,5 et 2,7.

21. Procédé selon la revendication 14, comprenant en outre la transformation de l'acide 1,1-cyclohexanediacétique en l'anhydride correspondant.

22. Procédé selon la revendication 21, dans lequel la transformation de l'acide 1,1-cyclohexanediacétique en l'anhydride correspondant est réalisée par réaction avec de l'anhydride acétique en présence d'un solvant organique.

23. Procédé selon la revendication 22, dans lequel le solvant organique est le toluène.
